# EUROPEAN PATENT SPECIFICATION

(11) **EP 1 279 402 B1**
(45) Date of publication and mention of the grant of the patent: **29.11.2006**
(21) Application number: 01402027.5
(22) Date of filing: 26.07.2001
(51) Int. Cl.: A61K 9/20, A61K 9/16

(54) **Coated granules of allylamine-or benzylamine-anti-mycotics, process for preparation thereof and orodispersible tablets containing said coated granules**
Umgehüllte Allylamine oder Benzylamine enthaltende Granulate, Verfahren zur Herstellung und in der Mundhöhle dispergierbare Tabletten enthaltend die umgehüllten Granulate
Granules enrobés d'allylamines ou de benzlyamines antimycotiques, procédé de préparation, et comprimés orodispersibles contenant les granules enrobés

(43) Date of publication of application: 29.01.2003
(73) Proprietor: ETHYPHARM, 78550 Houdan (FR)
(72) Inventor: Cousin, Gérard, Villemeux/Eure 28210 (FR); Bruna, Etienne, 28300 Jouy (FR); Gendrot, Edouard, 28500 Garnay (FR)
(74) Representative: Touati, Catherine

(56) References cited:
- WO-A-00/44353
- WO-A-00/51568
- WO-A-01/52895
- US-A- 6 136 344

## Description

The present invention concerns coated granules of an allylamine-or benzylamine-anti-mycotic, a process for the preparation thereof and orodispersible tablets comprising coated granules of an allylamine or benzylamine-anti-mycotic.

In the context of the present invention, the term "orodispersible tablets" means tablets which are able to disintegrate in the buccal cavity in less than 60 seconds, preferably in less than 40 seconds, upon contact with saliva by formation of an easy-to-swallow suspension.

Butenafine, naftifine and terbinafine are well known synthetic antimycotics. Terbinafine and naftinine are examples of allylamine-anti-mycotics and butenaftine is an example of benzylamine-anti-mycotic.

Terbinafine is known from e.g. EP-A-24587. It is acknowledged in the art and is commercially available, in particular as a swallowable tablet, under the trade name Lamisil®.

Allylamine- and benzylamine-anti-mycotics are highly active via both topical and oral administration. While numerous pharmaceutical compositions for topical and oral administration have been proposed, there still exists a need for commercially acceptable allylamine or benzylamine-anti-mycotic formulations for oral administration with good patient convenience and acceptance, especially for children or the elderly.

One particular difficulty in the formulation of allylamine- or benzylamine-anti-mycotics in oral pharmaceutical compositions is their unpleasant, bitter, taste and low physical integrity.

Furthermore, some patients, especially children and the elderly, experience difficulties swallowing the tablets, even with liquids. It is estimated that 50% of the population has problems swallowing the tablets. Said problem leads to poor or even noncompliance with the treatment and thus has a negative impact on the efficiency of the treatment (H. Seager, 1998, J. Pharm. Pharmacol. 50, 375-382).

Oral disintegrable multiparticulate tablets have already been described in EP 548356, EP1003484, WO 00/27357 and WO00/51568. The active ingredient is in the form of coated microcrystals or coated microgranules.

Up to now, no oral formulations of allylamine- or benzylamine-anti-mycotic exist which are specifically suitable for patients having difficulties when swallowing or for patients taking the drugs with no liquids.

It is thus highly desirable to remedy this situation and to develop an orodispersible tablet containing an allylamine- or benzylamine-anti-mycotic, which has taste-masking properties and presents a pleasant palatability such that the administration of the tablet is not unpleasant for the patient.

The Applicant has now surprisingly found that these characteristics can be obtained by formulating a tablet containing an allylamine-or benzylamine-anti-mycotic as active principle in the form of coated granules, and a mixture of excipients containing at least one disintegration agent, a soluble agent and a lubricant, and optionally a swelling agent, a permeabilising agent, sweeteners and flavouring agents.

Such a tablet allows the obtaining of pharmacokinetical parameters at least equivalent to those which are obtained with conventional oral formulations of allylamine- or benzylamine-anti-mycotic, especially terbinafine.

Surprisingly, although the tablets according to the invention disintegrate in the buccal cavity and present a liberation of the active principle which is equivalent to the conventional formulation, they nevertheless have a pleasant taste.

Furthermore, these rapidly disintegrating compositions of the invention were found to show high stability and physical integrity, e.g. during storage, handling, packaging and the like, without limiting the disintegration performance of the composition.

The tablets according to the invention comprise coated granules of an allylamine- or benzylamine- anti-mycotic, or one of its pharmaceutically acceptable salts.

The allylamine- or benzylamine-anti-mycotics, such as butenafine, naftifine and terbinafine may be used e.g. in free base form or in acid addition salt form. An acid addition salt form may be prepared from the free base form in a conventional manner and vice-versa. Examples of suitable acid addition salt forms include hydrochloride, lactate and ascorbate, preferably hydrochloride.

The present invention also relates to said coated granules.

The taste-masking of the allylamine- or benzylamine-anti-mycotic is achieved by coating, granulated microcrystals of the allylamine- or benzylamine-anti-mycotic with one or more polymers.

According to an advantageous embodiment of the invention, the granules of the allylamine- or benzylamine-anti-mycotic, or one of its pharmaceutically acceptable salts, are characterized in that the granules are coated and that they contain:
- microcrystals of the allylamine- or benzylamine-anti-mycotic, or one of its pharmaceutically acceptable salts,
- at least one binder,
- optionally a diluent agent and/or an antistatic agent, and/or a permeabilising agent.

The binder is selected from the group consisting of cellulosic polymers, acrylic polymers, povidones, polyvinylalcohols and mixtures thereof, preferably polyvinylpyrrolidone (Kollidon K30).

Optionally, in order to enhance the granulation of the allylamine-or benzylamine-anti-mycotic or one of its pharmaceutically acceptable salts, a diluent agent is used.

The diluent agent is selected from the group comprising cellulosic derivatives and is preferably selected from the group consisting of microcrystalline cellulose, starches, lactose and polyols such as mannitol.

In order to facilitate the fluidification of the composition during the granulation step, it is possible to use a permeabilising agent, and/or an antistatic agent.

Said permeabilising agent is selected from the group comprising colloidal silica (Aerosil®) precipitated silica (Syloïd® FP244).

Said antistatic agent is selected from the group of micronised or non micronised talc.

According to another embodiment of the invention, the granules comprise :
- from 10% to 80% of granules of an allylamine- or benzylamine-anti-mycotic, or one of the pharmaceutically acceptable salts thereof,
- at most 15%, preferably at most 5% by weight of the binder, relative to the weight of the allylamine- or benzylamine-anti-mycotic, or one of the pharmaceutically acceptable salts thereof,
- at most 2%, preferably 0.5% by weight of the antistatic agent, relative to the weight of the granules of allylamine- or benzylamine-anti-mycotic, or one of the pharmaceutically acceptable salts thereof.

In order to ensure efficient taste masking, and a dissolution profile of the active substance such that more than 80% of the active substance is released in 30 minutes, preferably 70% is released in 15 minutes, the granules are coated with a coating composition containing at least one coating polymer selected from the group consisting of cellulosic polymers, acrylic polymers and their mixtures.

Among the cellulosic polymers, ethylcellulose, hydroxypropylcellulose (HPC) and hydroxypropylmethylcellulose (HPMC), are advantageously used.

Among the acrylic polymers, insoluble acrylate ammonio-methacrylate copolymer (Eudragit®RL100 or RS100 or Eudragit® RL30D or RS30D), polyacrylate (Eudragit® NE), or methacrylic copolymers (Eudragit® L100-55 or Eudragit® L30D), are advantageously used, alone, in combination or in admixture with pH-dependant polymers. A mixture of Eudragit® RL100 with Eudragit®L100-55 is preferred, with a ratio comprised respectively of between 70/30 and 30/70, more preferably 30/70.

The prepared coating liquid is either water-based or prepared with organic solvents. According to an advantageous embodiment, this coating liquid is suitable to be sprayed with conventional spray layering equipment.

Optionally a permeabilising agent, plasticizers, and soluble agents are added as coating additives.

The plasticizer is selected in the group consisting of triacetine, triethylacetate, triethylcitrate (Eudraflex®), ethyl phthalate, or mixtures thereof. The plasticizer is used in proportions of at most about 30%, preferably 10% by weight of the coating polymers.

The soluble agents are selected in particular among the polyols.

Advantageously the coated granules according to the invention present a granulometric repartition such that at least 50%, preferably at least 70% of the granules have a particle size ranging between 125 and 355µm and less than 15% of the granules have a particle size less than 90µm.

The particle sizes are measured according to conventional methods, preferably by sieving.

In a particular embodiment, the coated granules according to the invention comprise:
- from 10% to 95% of granules of the allylamine- or benzylamine-anti-mycotic, or one of its pharmaceutically acceptable salts, preferably terbinafine HCI,
- from 5 to 60%, preferably 5 to 40% and even more preferably from 10 to 20% of a coating polymer, preferably a mixture of Eudragit RL100/Eudragit L100-55, the percentages being expressed by weight relative to the weight of the granules of the allylamine- or benzylamine-anti-mycotic, or one of its pharmaceutically acceptable salts,
- from 0 to 30%, preferably 10% by weight of plasticizers, preferably ethyl phthalate, the percentages being expressed by weight relative to the weight of the coating polymer,
- from 0 to 10% of a permeabilising agent, preferably colloidal silica, the percentages being expressed by weight relative to the weight of the coating polymer, and
- from 0 to 10% of an antistatic agent, preferably micronised talc, the percentages being expressed by weight relative to the weight of the coating polymer.

According to another embodiment of the invention, the coated granules of the allylamine or benzylamine-anti-mycotic, or one of its pharmaceutically acceptable salts, further comprise an overcoating which consists essentially in acrylic polymer, cellulosic polymer, thermoplastic polymer or mixtures thereof.

Optionally the overcoating ratio is from about 10 to about 60% by weight relative to the coated granules.

The invention also concerns a process for preparing coated granules of the allylamine- or benzylamine-anti-mycotic, or one of its pharmaceutically acceptable salts.

The process comprises the successive steps consisting in :
- dry mixing the microcrystals of the allylamine- or benzylamine-anti-mycotic, or one of its pharmaceutically acceptable salts optionally with an antistatic agent and/or a diluent agent, and/or a permeabilising agent;
- granulating the thus obtained mixture by spraying thereon a solution or a suspension of the binder,
- coating the thus obtained granules by spraying thereon a suspension, a dispersion or a solution of the coating composition,
- optionally, applying an overcoating based on acrylic polymer, cellulosic polymer, thermoplastic polymer or mixtures thereof,
- drying the thus obtained coated granules.

In this process the mixing, granulating and coating steps can be performed in different apparatuses or in the same apparatus, each step being performed in the presence of a mixture of excipients which are identical or different.

In an advantageous embodiment, each step is performed on a fluidized air-bed, such as for example, but not limited to Glatt GPCG-1, GPCG-5 or GPCG 120.

For granulating, bottom spray granulation, tangential spray granulation, top spray granulation or high shear granulation can be used, the bottom spray granulation being preferred.

For coating, bottom, top and tangential spray methods can be used as well as layering method, bottom spray method of coating being preferred.

The optional overcoating step can be performed using the same method as used for the coating step.

The coated granules thus obtained are particularly useful for incorporation in the manufacture of rapidly disintegratable tablets, which are also another object of the present invention.

The present invention also relates to orodispersible tablets, i.e. rapidly disintegratable tablets which are able to disintegrate in the buccal cavity upon contact with saliva by formation of an easy-to-swallow suspension, in less than 60 seconds, preferably in less than 40 seconds. Said tablets are characterized by the fact that they contain the above described coated granules, and a mixture of excipients comprising at least one disintegrating agent, a soluble diluent agent, a lubricant and optionally a swelling agent, a permeabilising agent, sweeteners, flavoring agents and colors.

The disintegration time corresponds to the time between the moment when the tablet is placed in the buccal cavity in contact with saliva and the moment when the suspension resulting from the disintegration without chewing of the tablet is swallowed.

According to an advantageous embodiment, the tablet according to the invention, have a hardness of not less than 15 N, when measured with the test method of the European Pharmacopeia (2.9.8)

According to an advantageous embodiment, the tablet according to the invention, contains coated granules of an allylamine- or benzylamine-anti-mycotic, or one of its pharmaceutically acceptable salts, and a mixture of excipients, the ratio of the mixture of excipients to the coated granules is 0.4 to 10, preferably 0.4 to 5 and even more preferably 1 to 2 parts by weight, the mixture of excipients comprising:
- at least one disintegrating agent,
- a soluble diluent agent which presents binding properties,
- a lubricant,
- a permeabilising agent,
- and optionally sweeteners, flavorings and colors.

The disintegrating agents are selected from the group consisting of croscarmellose, available as e.g. Ac-di-sol®, crospovidone available as e.g. Kollidon CL®, and mixtures thereof.

The soluble diluent agent with binding properties consists of a polyol having less than 13 carbon atoms and being either in the form of the directly compressible product with an average particle size of 100 to 500 µm, or in the form of a powder with an average particle size of less than 100 µm, this polyol preferably being selected from the group comprising mannitol, xylitol, sorbitol and maltitol, it being understood that sorbitol cannot be used alone and that, in the case where there is only one soluble diluent agent with binding properties, it is used in the form of the directly compressible product, whereas in the case where there are at least two soluble diluent agents with binding properties, one is present in the directly compressible form and the other is present in powder form, it then being possible for the polyols to be the same, the ratio of directly compressible polyol to powder polyol being 99/1 to 20/80, preferably 80/20 to 20/80.

The proportion of disintegrating agents being 1 to 20% by weight, preferably 5 to 15% by weight, in the case of a mixture, each disintegrating agent being comprised between 0.5 and 15% by weight, preferably 3 to 8 % by weight, and the proportion of soluble agent being 30 to 90% by weight, preferably 30 to 50% by weight, based in each case on the weight of the tablet.

The lubricant is selected from the group consisting of magnesium stearate, stearic acid, sodium stearyl fumarate, micronised polyoxyethyleneglycol (micronised Macrogol 6000), leukine, sodium benzoate and mixtures thereof.

The amount of lubricant is from 0.02 to 2 percent, preferably from 0.5 to 1 percent (weight of the lubricant /total weight of the tablet).

The lubricant can be dispersed within the mixture of excipients, or according to an advantageous embodiment, it can be dispersed on the surface of the tablet.

The permeabilizing agent used is a compound selected from the group comprising especially silicas with a high affinity for aqueous solvents, such as the precipitated silica better known by the trademark Syloïd®^{,} maltodextrins, β-cyclodextrins and mixtures thereof.

The permeabilizing agent allows the creation of a hydrophilic network which facilitates the penetration of saliva and hence assists the disintegration of the tablet.

The proportion of permeabilising agent is between 0.5 and 5% by weight, based on the weight of the tablet.

A sweetener and optionally a flavoring and a color are also included in the mixture of excipients forming part of the composition of the tablets according to the invention.

The sweetener can be selected from the group comprising especially aspartame, potassium acesulfame, sodium saccharinate, neohesperidin dihydrochalcone, sucralose, monoammonium glycyrrhizinate, and mixtures thereof.

The flavorings and colors are those conventionally used in pharmacy for the preparation of tablets.

Determination of workable proportions in any particular instance will generally be within the capability of the man skilled in the art. All indicated proportions and relative weight ranges described above are accordingly to be understood as being indicative of preferred or individually inventive teachings only and not as limiting the invention in its broadest aspect.

Details concerning any of the excipients of the invention may be found in Fiedler, H. P. "Lexikon der Hilfsstoffe für Pharmazie, Kosmetik und angrenzende Gebiete", Editio Cantor Verlag Aulendorf, Aulendorf, 4th revised and expanded edition (1996); "Handbook of Pharmaceutical Excipients", 2nd Edition, Editors A. Wade and P. J. Weller (1994), Joint publication of American Pharmaceutical Association, Washington, USA and The Pharmaceutical Press, London, England; or may be obtained from the relevant manufacturers,

For compressing the mixture of coated granules and excipients into a tablet, various punches may be used, with diameters comprised between 10 and 14 mm, depending upon the dosage of the tablet.

Various shapes may be used, such as for example, flat shape, advantageously with bevelled edges or polo punches.

The orodispersible tablets of the present invention show fast disintegration in the buccal cavity upon contact with saliva without chewing, in less than 60 seconds, preferably in less than 40 seconds, have a pleasant taste and palatability and thus have particularly good patient convenience and patient acceptance due to their increased ease of administration and ingestion.

In addition the tablets of the invention show surprisingly high physical stability and are easy to handle and package.

The tablets of this invention are useful for the known indications of the allylamine- or benzylamine-anti-mycotics, e.g. for the following conditions: onychomycosis caused by dermatophyte fungi, tinea capitis, fungal infections of the skin, for the treatment of tinea corporis, tinea cruris, tinea pedis, and yeast infections of the skin caused by the genus Candida, e.g. Candida albicans, systemic mycosis, mycosis by azole-resistant strains, e.g. in combination with a 14-alpha-methyldimethylase inhibitor, or infections with Helicobacter pylori.

The tablets are particularly effective in treating tinea capitis in e.g. children.

The utility of the tablets of the present invention may be observed in standard bioavailability tests or standard animal models, for example ascertaining dosages of the present tablets giving blood levels of the allylamine- or benzylamine-anti-mycotic, expressed as allylamine- or benzylamine-anti-mycotic base equivalent to blood levels giving a therapeutical effect on administration of known allylamine- or benzylamine-anti-mycotic oral dosage forms, e.g. a tablet. typical doses are in the range of 1 mg/kg to 10 mg/kg, e.g. 1.5 mg/kg to 5 mg/kg, or e.g. 3 to 4 mg/kg body weight of allylamine- or benzylamine-anti-mycotic per day.

The appropriate dosage will, of course, vary depending upon, for example, the host and the nature and severity of the condition being treated. However, in general satisfactory results in animals are indicated to be obtained at daily treatments with doses from about 1 mg/kg to about 10 mg/kg animal body weight. In humans an indicated daily dosage is in the range from about 10 mg to about 1000 mg per day, conveniently administered, for example, in divided doses up to four times a day or once daily. Preferred dosages, expressed as allylamine- or benzylamine-anti-mycotic base, for children weighing less than 20 kg may be about 62.5 mg once daily, for children weighing 20 to 40 kg about 125 mg once daily, for children weighing more than 40 kg about 250 mg once daily, and for adults from about 250 mg to about 500 mg once daily.

The invention is illustrated more in detail in the following examples.

### EXAMPLES

In the examples below, the following products are used:
- HPMC: hydroxypropylmethylcellulose
- methacrylic copolymers (Eudragit®L30D-55, Eudragit® E100, Eudragit® L100D-55, sold by Röhm),
- Kollidon 30: polyvinylpyrrolidone sold by BASF,
- Aquacoat: ethylcellulose sold by FMC,
- Ac-Di-Sol: sodium croscarmellose, sold by FMC,
- Kollidon CL: crospovidone, sold by BASF,
- Eudraflex: triethylcitrate, sold by Röhm,
- Syloïd 244 FP: precipitated silica.

### Example 1 :

### Granulating step

- 400g of terbinafine HCI were granulated in a fluidized bed GPCG1 GLATT with 1432g of an aqueous suspension including 80g of HPMC and 400g of terbinafine HCI, by spraying onto the powder.

### Coating step

The thus obtained granules were coated by spraying thereon 12986g of an aqueous suspension containing 400g of Eudragit® L30D-55 and 120g of Eudragit® E100, at a pH of not less than 2.5.

The amount of coating was of 20% by weight with respect to the weight of the granules of terbinafine HCI for example 1A and of 30% by weight with respect to the weight of the granules of terbinafine HCI for example 1B.

The dissolution rates of said granules were measured as follows:

An amount of granules corresponding to 125mg of terbinafine base was added once to 500ml of the European pharmacopoeia buffer of pH=3, under stirring at 50rpm. A sample of the solution was analyzed by HPLC at 280nm, at t=2, 5, 10, 15 and 30 minutes, in order to determine the percentage of dissolved terbinafine.

The results are given in the following table 1.:

**Table 1**

| | Coated granules of example 1A | Coated granules of example 1B |
|---|---|---|
| 2 minutes | 41.8% | 23.3% |
| 5 minutes | 75.8% | 62.7% |
| 10 minutes | 90.8% | 84.8% |
| 15 minutes | 95.4% | 92.1% |
| 30 minutes | 96.8% | 95.3% |

After 30 minutes, more than 80% of terbinafine was dissolved, however the taste was not very pleasant, due to a non compatibility between Eudragit® E100 and terbinafine HCI.

### Example 2:

### Granulating step

- 800g of terbinafine HCI were granulated in a fluidized bed GPCG1 with 720g of ethanol in which 80g of polyvinylpyrrolidone was dissolved.

### Coating step:

The thus obtained granules were coated by spraying thereon an homogeneous mixture of 720g of HPMC in water (10%w/w) and 593,6g of a mixture of Aquacoat ECD30 and triethylcitrate (20% by weight of polymer).

The amount of coating was of 20% by weight with respect to the weight of the granules of terbinafine HCI for example 2A and of 30% by weight with respect to the weight of the granules of terbinafine HCI for example 2B.

The dissolution rates of said granules were measured as indicated in example 1 above.

The results are given in the following table 2.:

**Table 2**

| | Coated granules of example 2A | Coated granules of example 2B |
|---|---|---|
| 2 minutes | 25.8% | 25.7% |
| 5 minutes | 54.9% | 53.8% |
| 10 minutes | 81.6% | 82.3% |
| 15 minutes | 91.5% | 92.3% |
| 30 minutes | 97.6% | 99.3% |

More than 80% of terbinafine is dissolved after 30 minutes, taste-masking is efficient.

### Example 3:

### Granulating step:

- 880g granules of terbinafine HCI were prepared as described in example 1 (granulating step).

### Coating step:

The thus obtained granules were coated by spraying thereon 1840 g of homogeneous dispersion containing 720g of HPMC in water (10%w/w) and 1120g of Eudragit® L30D.

The amount of coating was of 20% by weight with respect to the weight of the granules of terbinafine HCI for example 3A and of 30% by weight with respect to the weight of the granules of terbinafine HCI for example 3B.

The dissolution rates of said granules were measured as indicated in example 1 above.

The results are given in the following table 3:

**Table 3**

| | Coated granules of example 3A | Coated granules of example 3B |
|---|---|---|
| 2 minutes | 24.2% | 18.8% |
| 5 minutes | 59.1% | 47.6% |
| 10 minutes | 78.5% | 76.7% |
| 15 minutes | 88.6% | 89.2% |
| 30 minutes | 94.9% | 97.8% |

More than 80% of terbinafine is dissolved after 30 minutes.

### Example 4:

- 500g of mannitol were introduced in a fluidized bed GPCG-1
- 1000g of terbinafine HCI were suspended with 1000g of ethanol and 2000g of acetone
- 300g of PVP K90 were then dissolved in the suspension. The obtained suspension was sprayed onto the mannitol cores.

### Coating step

The thus obtained granules were coated by spraying thereon a mixture of the same composition as for example 3 (granulating step).

The amount of coating was of 30% by weight with respect to the weight of the granules of terbinafine HCI.

The dissolution rate of said granules was measured as indicated in example 1 above.

The results are given in the following table 4.:

**Table 4**

| | Coated granules of example 4 |
|---|---|
| 2 minutes | 30.2% |
| 5 minutes | 56.9% |
| 10 minutes | 81.2% |
| 15 minutes | 89.9% |
| 30 minutes | 93.0% |

More than 80% of terbinafine HCI is dissolved after 30 minutes.

### Example 5:

### Granulation step

- Granulation of 800 g of terbinafine HCI was prepared as in example 1 (granulating step)

### Coating step

The thus obtained granules were coated by spraying thereon 1392g of an aqueous dispersion of Eudragit® L30D and Eudragit®RL30D, respectively in a ratio of (70/30), with triacetine as plasticizer.

The amount of coating was of 30% by weight with respect to the weight of the granules of terbinafine HCI.

The dissolution rates of said granules were measured as indicated in example 1 above.

The results are given in the following table 5.

**Table 5**

| | Coated granules of example 5 |
|---|---|
| 2 minutes | 19,1 |
| 5 minutes | 41,4 |
| 10 minutes | 63,3 |
| 15 minutes | 75,5 |
| 30 minutes | 89,2 |

More than 80% of terbinafine is dissolved after 30 minutes, taste-masking is effective.

### Example 6:

Tablets according to the invention were prepared as follows.

### Granulation step:

**Table 6a**

| **Components** | **Quantity (g)** | **%** |
|---|---|---|
| terbinafine HCI | 8000 | 94.79 |
| Kollidon K30 | 400 | 4.74 |
| Syloïd 244 FP | 40 | 0.47 |
| Ethanol USP/PE | 3600 | |

The components given in Table 6a above were introduced in a fluidized bed . During the granulation the temperature was maintained between 19 to 22°C.

### Coating step:

The obtained granules were then coated with the coating composition given in table 6b below:

**Table 6b**

| **Components** | **Amount (g)** | **%** |
|---|---|---|
| Granules of terbinafine HCI | 8000 | 65.27 |
| Eudragit® L100-55 | 2128 | 17.36 |
| Eudragit® RL100 | 912 | 7.44 |
| Ethyl phthalate | 304 | 2.48 |
| Syloïd 244 FP | 912 | 7.44 |
| Ethanol USP/PE | 3600 | |

The coating ratio was 38% by weight with respect to the weight of the granules of terbinafine HCI.

Said coated granules present a good dissolution rate and a good taste.

### Tabletting step:

The components listed in Table 6c below were homogeneously mixed.

The homogeneous obtained mixture was introduced in a tabletting machine, with a tabletting speed of 60000 tablets/hour. The punches used were 12 mm polo punches. The internal wall of the punches were pulverized with magnesium stearate and then 400.00mg of the mixture were introduced in each punch.

**Table 6c**

| **Components** | **Unit composition (mg)** |
|---|---|
| terbinafine HCI coated granules | 230.16* |
| Pearlitol 400 DC | 104.84 |
| Kollidon CL | 25.00 |
| AcDisol | 20.00 |
| Aspartam | 8.00 |
| Tutti Frutti flavor | 9.00 |
| Syloïd FP 244 | 3.00 |
| Magnesium stearate | qsp lubricating |
| Total weight of tablets | 400.00 |

| | |
|---|---|
| *corresponds to 125 mg terbinafine base. | |

- The hardness of the tablets was 39N
- Thickness: 4.52mm.
- Disintegration time: less than 30s.
- The taste was pleasant.

### Example 7

The granules obtained in example 6 above (granulation step) were coated at a coating amount of 25% and of 30%

The respective coating compositions are given in table 7b below:

**Table 7b**

| | Coating ratio 25% | | Coating ratio 30% | |
|---|---|---|---|---|
| Components | Amount (g) | % | Amount (g) | % |
| Granules of terbinafine HCI | 8263.0 | 74.07 | 8201.0 | 94.64 |
| Eudragit® L100-55 | 1446.0 | 12.96 | 232.5 | 2.68 |
| Eudragit® RL100 | 619.7 | 5.56 | 99.6 | 1.15 |
| Ethyl phthalate | 206.6 | 1.85 | 33.2 | 0.38 |
| Syloïd 244 FP | 619.7 | 5.56 | 99.6 | 1.15 |
| Ethanol USP/PE | 14460 | | 2325 | |

Said coated granules present a good dissolution rate and a good taste.

### Tabletting step:

The components listed in Table 7c below were homogeneously mixed.

The homogeneous obtained mixture was introduced in a tabletting machine, with a tabletting speed of 50000 tablets/hour. The punches used were 12 mm flat punches with bevelled edges. The internal wall of the punches were pulverized with magnesium stearate and then 400.00mg of the mixture were introduced in each punch.

**Table 7c**

| | Coating amount of 25% | Coating amount of 30% |
|---|---|---|
| Components | Unit composition (mg) | Unit composition (mg) |
| terbinafine HCI coated granules | 200.39* | 210.78* |
| Pearlitol 400 DC | 134.61 | 124.22 |
| Kollidon CL | 25.00 | 25.00 |
| AcDisol | 20.00 | 20.00 |
| Aspartam | 8.00 | 8.00 |
| Tutti Frutti flavor | 9.00 | 9.00 |
| Syloïd FP 244 | 3.00 | 3.00 |
| Magnesium stearate | qsp lubricating | qsp lubricating |
| Total weight of tablets | 400.00 | 400.00 |

| | | |
|---|---|---|
| *correspond to 125 mg terbinafine base. | | |

- The hardness of the tablets was respectively 36N and 44N.
- Thickness was: 4.30mm in each case.
- Disintegration time: less than 30s.
- The taste was pleasant

### Exemple 8

The granules obtained in example 6 above (granulation step) were coated at a coating amount of 15% and of 10%

The respective coating compositions are given in table 8b below:

**Table 8b**

| | Coating amount of 15% | | Coating amount of 10% | |
|---|---|---|---|---|
| Components | Amount (g) | % | Amount (g) | % |
| terbinafine granules | 6572.0 | 93.87 | 8000.0 | 87.62 |
| Eudragit® L100-55 | 214.7 | 3.07 | 560.0 | 6.14 |
| Eudragit® RL100 | 92.0 | 1.31 | 240.0 | 2.63 |
| Ethyl phthalate | 30.7 | 0.44 | 80.0 | 0.88 |
| Syloïd 244 FP | 92.0 | 1.31 | 240.0 | 2.63 |
| Ethanol USP/PE | 2146.7 | | 5600 | |

Said coated granules present a dissolution rate of more than 80% after 30 mn.

### Tabletting step:

The components listed in Table 8c below were homogeneously mixed.

The homogeneous obtained mixture was introduced in a tabletting machine, with a tabletting speed of 50000 tablets/hour. The punches used were 12 mm flat punches with bevelled edges. The internal wall of the punches were pulverized with magnesium stearate and then 400.00mg of the mixture were introduced in each punch.

**Table 8c**

| | Coating ratio of 15% | Coating ratio of 10% |
|---|---|---|
| Components | Unit composition (mg) | Unit composition (mg) |
| terbinafine coated granules | 180.16* | 169.12* |
| Pearlitol 400 DC | 154.84 | 165.88 |
| Kollidon CL | 25.00 | 25.00 |
| AcDisol | 20.00 | 20.00 |
| Aspartam | 8.00 | 8.00 |
| Tutti Frutti flavor | 9.00 | 9.00 |
| Syloïd FP 244 | 3.00 | 3.00 |
| Magnesium stearate | qsp lubricating | qsp lubricating |
| Total weight of tablets | 400.00 | 400.00 |

| | | |
|---|---|---|
| *correspond to 125 mg terbinafine base. | | |

- The hardness of the tablets was respectively 35 and 38 N
- Disintegration time: less than 30s.
- The taste was pleasant

## Claims

1. Granules of allylamine- or benzylamine-anti-mycotic, or one of its pharmaceutically acceptable salts, **characterized in that** the granules are coated and that they contain:
- microcrystals of an allylamine- or benzylamine-anti-mycotic or one of its pharmaceutically acceptable salts,
- at least one binder selected from the group consisting of cellulosic polymers, such as ethylcellulose, hydroxypropylcellulose and hydroxypropylmethylcellulose, acrylic polymers such as insoluble acrylate ammoniomethacrylate copolymer, polyacrylate or polymethacrylic copolymer; povidones, polyvinylalcohols and mixtures thereof,
- optionally a diluent agent selected from the group comprising cellulosic derivatives, microcrystalline cellulose, starches, lactose, polyols such as mannitol and/or an antistatic agent selected from the group comprising micronised or non micronised talc, and/or a permeabilising agent selected from the group consisting of colloidal silica and precipitated silica.

2. Granules according to claim 1, **characterized in that** the allylamine- or benzylamine-anti-mycotic is selected from the group consisting of butenafine, naftifine, and preferably terbinafine.

3. Granules according to claim 1 or claim 2, **characterized in that** they present a granulometric repartition such that at least 50%, preferably at least 70%, of the granules have a particle size ranging between 125 and 355µm and less than 15% of the granules have a particle size less than 90µm.

4. Granules according to anyone of claims 1 to 3, **characterized in that** they comprise:
- from 10% to 80% of granules of the allylamine- or benzylamine-anti-mycotic, or one of its pharmaceutically acceptable salts, relative to the weight of the granules of the allylamine- or benzylamine-anti- mycotic, or one of its pharmaceutically acceptable salts,
- at most 15%, preferably at most 5% by weight of the binder, relative to the weight of the allylamine- or benzylamine-anti-mycotic, or one of its pharmaceutically acceptable salts,
- at most 2%, preferably 0.5% by weight of the antistatic agent, relative to the weight of the granules of the allylamine- or benzytamine-anli-mycotic, or one of its pharmaceutically acceptable salts.

5. Granules according to any one of claims 1 to 4, **characterized by** the fact that they are coated with a coating composition containing at least one coating polymer selected from the group consisting of cellulosic polymers such as ethylcellulose, hydroxypropylcellulose and hydroxypropylmethylcellulose, acrylic polymers such as insoluble acrylate ammonio-methacrylate copolymer, polyacrylate, or methacrylic copolymers used, alone, in combination or in admixture with pH-dependant polymers, and their mixtures, and optionally a permeabilising agent, plasticizers, and soluble agents such as polyols.

6. Granules according to any one of claims 1 to 5, **characterized by** the fact that they comprise:
- from 10% to 95% of granules of allylamine- or benzylamine-anti-mycotic or one of its pharmaceutically acceptable salts, preferably terbinafine HCl, the percentages being expressed by weight relative to the weight of the granules of the allylamine- or benzylamine-anti-mycotic, or one of its pharmaceutically acceptable salts,
- from 5 to 60%, preferably from 5 to 40% and even more preferably from 10 to 20% of a coating polymer, the percentages being expressed by weight relative to the weight of the granules of the allylamine- or benzylamine-anti-mycotic or one of its pharmaceutically acceptable salts,
- from 0 to 30%, preferably 10% by weight of plasticizers, preferably ethyl phthalate, the percentages being expressed by weight relative to the weight of the amount of the coating polymer,
- from 0 to 10% of a permeabilising agent, preferably colloidal silica, the percentages being expressed by weight relative to the weight of the amount of the coating polymer, and
- from 0 to 10% of an antistatic agent, preferably micronised talc the percentages being expressed by weight relative to the weight of the amount of the coating polymer.

7. Granules according to any one of claims 1 to 6, **characterized by** the fact that they further comprise an overcoating essentially consisting in acrylic polymer, cellulosic polymer, thermoplastic polymer or mixtures thereof.

8. Process for the preparation of granules according to any one of claims 1 to 7, **characterized by** the fact that it comprises the successive steps consisting in:
- dry mixing the microcrystals of the allylamine- or benzylamine-anti-mycotic or one of its pharmaceutically acceptable salts optionally with an antistatic agent and/or a diluent agent, and/or permeabilising agent;
- granulating the mixture obtained in the above step by spraying of a solution or suspension of the binder,
- coating the thus obtained granules with a suspension of the coating composition,
- optionally, applying onto the thus obtained coated granules an overcoating based on acrylic polymer, cellulosic polymer, thermoplastic polymer or mixtures thereof,
- drying the thus obtained coated granules.

9. Process according to claim 8, wherein the granulation is a bottom spray granulation, tangential spray granulation, top spray granulation or high shear granulation, preferably bottom spray granulation.

10. Orodispersible tablets comprising coated granules according to anyone of claims 1 to 7 or prepared according to claim 8 or 9, and a mixture of excipients comprising at least one disintegrating agent, a soluble diluent agent, a lubricant and optionally a swelling agent, a permeabilising agent, sweeteners, flavorings and colors.

11. Orodispersible tablets according to claim 10, in which the ratio of the mixture of excipients to the coated granules is 0.4 to 10, preferably 0.4 to 5 and even more preferably 1 to 2 parts by weight, the mixture of excipients comprising:
- at least one disintegrating agent,
- a soluble diluent agent which presents binding properties.
- a lubricant,
- a permeabilising agent,
- and optionally sweeteners, flavorings and colors.

12. Orodispersible tablets according to claim 10 or 11, in which the disintegrating agent is selected from the group consisting of croscarmellose, crospovidone and mixtures thereof.

13. Orodispersible tablets according to anyone of claims 10 to 12 in which the soluble diluent agent with binding properties consists of a polyol having less than 13 carbon atoms and being either in the form of the directly compressible product with an average particle size of 100 to 500 µm, or in the form of a powder with an average particle size of less than 100 µm, this polyol preferably being selected from the group consisting of mannitol, xylitol, sorbitol, and maltitol, it being understood that sorbitol cannot be used alone and that, in the case where there is only one soluble diluent agent with binding properties, it is used in the form of the directly compressible product, whereas in the case where there are at least two soluble diluent agents with binding properties, one is present in the directly compressible form and the other is present in powder form, it then being possible for the polyols to be the same, the ratio of directly compressible polyol to powder polyol being 99/1 to 20/80, preferably 80/20 to 20/80.

14. Orodispersible tablets according to anyone of daims 10 to 13, in which the permeabilizing agent is selected from the group comprising especially silicas with a high affinity for aqueous solvents, such as the precipitated silica better known by the trade mark Syloïd^{®}, maltodextrins, β-cyclodextrins and mixtures thereof.

15. Orodispersible tablets according to anyone of claims 10 to 14, in which the lubricant is selected from the group consisting of magnesium stearate, stearic acid, sodium stearyl fumarate, micronised polyoxyethyleneglycol (micronised Macrogol 6000), leukine, sodium benzoate and mixtures thereof.

16. Orodispersible tablets according to anyone of claims 10 to 15, in which the proportion of disintegrating agent is 1 to 20% by weight, preferably 5 to 15% by weight, and the proportion of soluble agent is 30 to 90% by weight, preferably 30 to 50% by weight, based in each case on the weight of the tablet.

17. Use of granules of allylamime- or benzylamine-anti-mycotic, or one of its pharmaceutically acceptable salts according to any one of claims 1 to 7 for the manufacture of orodispersable tablets according to any one of claims 10 to 16 for the following therapeutic applications: onychomycosis caused by dermatophyte fungi, tinea capitis, fungal infections of the skin, for the treatment of tinea corporis, tinea cruris, tinea pedis, and yeast infections of the skin caused by the genus Candida, e.g. Candida albicans, systemic mycosis, mycosis by azole-resistant strains, e.g. in combination with a 14-alpha-methyldimethylase inhibitor, or infections with Helicobacter pylori.

## Patentansprüche

1. Granulat eines Allylamin- oder Benzylamin-Antimykotikums oder eines pharmazeutisch annehmbaren Salzes davon, **dadurch gekennzeichnet, dass** das Granulat beschichtet ist und enthält:
• Mikrokristalle eines Allylamin- oder Benzylamin-Antimykotikums oder eines pharmazeutisch annehmbaren Salzes davon,
• wenigstens ein Bindemittel, ausgewählt aus der Gruppe bestehend aus Zellulose-Polymeren wie beispielsweise Ethylzellulose, Hydroxypropylzellulose und Hydroxypropylmethylzellulose, Acrylsäure-Polymeren wie beispielsweise unlösliches Acrylat-Ammoniummethacrylat-Copolymer, Polyacrylat oder Polymethacrylsäure-Copolymer; Povidone, Polyvinylalkohole und Gemische davon,
• gegebenenfalls ein Verdünnungsmittel, ausgewählt aus der Gruppe umfassend Zellulose-Derivate, mikrokristalline Zellulose, Stärken, Laktose, Polyole wie beispielsweise Mannitol und/oder ein Antistatikmittel, ausgewählt aus der Gruppe umfassend mikronisierten oder nicht-mikronisierten Talk und/oder ein Permeabilisierungsmittel, ausgewählt aus der Gruppe bestehend aus kolloidalem Siliciumdioxid und präzipitiertern Siliciumdioxid.

2. Granulat nach Anspruch 1, **dadurch gekennzeichnet, dass** das Allylamin- oder Benzylamin-Antimykotikum ausgewählt ist aus der Gruppe, bestehend aus Butenafin, Naftifin und bevorzugt Terbinafin.

3. Granulat nach Anspruch 1 oder Anspruch 2, **dadurch gekennzeichnet, dass** es eine solche Komgrößenverteilung aufweist, dass wenigstens 50%, bevorzugt wenigstens 70% des Granulats eine Teilchengröße im Bereich zwischen 125 und 355 µm aufweist und weniger als 15% des Granulats eine Teilchengröße von weniger als 90 µm aufweist.

4. Granulat nach einem der Ansprüche 1 bis 3, **dadurch gekennzeichnet, dass** es umfasst:
• 10 bis 80% Granulat des Allylamin- oder Benzylamin-Antimykotikums oder eines pharmazeutisch annehmbaren Salzes davon, bezogen auf das Gewicht des Granulats des Allylamin- oder Benzylamin-Antimykotikums oder eines pharmazeutisch annehmbaren Salzes davon,
• höchstens 15 Gew.-%, bevorzugt höchstens 5 Gew.-% Bindemittel, bezogen auf das Gewicht des Allylamin- oder Benzylamin-Antimykotikums, oder eines pharmazeutisch annehmbaren Salzes davon,
• höchstens 2 Gew.-%, bevorzugt 0.5 Gew.-% Antistatikmittel, bezogen auf das Gewicht des Granulats des Allylamin- oder Benzylamin-Antimykotikums, oder eines pharmazeutisch annehmbaren Salzes davon.

5. Granulat nach einem der Ansprüche 1 bis 4, **dadurch gekennzeichnet, dass** es mit einer Beschichtungszusammensetzung beschichtet ist, die wenigstens ein Beschichtungspolymer, ausgewählt aus der Gruppe bestehend aus Zellulose-Polymeren wie beispielsweise Ethylzellulose, Hydroxypropylzellulose und Hydroxypropylmethylzellulose, Acrylsäure-Polymeren wie beispielsweise unlösliches Acrylat-Ammoniummethacrylat-Copolymer, Polyacrylat oder Methacrylsäure-Copolymeren, die allein oder in Kombination oder im Gemisch mit pH-abhängigen Polymeren verwendet sind, und deren Gemischen, und gegebenenfalls ein Permeabilisierungsmittel, Weichmacher und lösliche Mittel wie beispielsweise Polyole beinhaltet.

6. Granulat nach einem der Ansprüche 1 bis 5, **dadurch gekennzeichnet, dass** es umfasst:
• 10% bis 95% Granulat eines Allylamin- oder Benzylamin-Antimykotikums oder eines pharmazeutisch annehmbaren Salzes davon, bevorzugt Terbinafin HCl, wobei die Prozentangaben in Gew.-%, bezogen auf das Gewicht des Granulats des Allylamin- oder Benzylamin-Antimykotikums, oder eines pharmazeutisch annehmbaren Salzes davon, ausgedrückt sind,
• 5 bis 60%, bevorzugt 5 bis 40% und noch weiter bevorzugt 10 bis 20% eines Beschichtungspolymers, wobei die Prozentangaben in Gew.-%, bezogen auf das Gewicht des Granulats des Allylamin- oder Benzylamin-Antimykotikums, oder eines pharmazeutisch annehmbaren Salzes davon ausgedrückt sind,
• 0 bis 30 Gew.-%, bevorzugt 10 Gew.-% Weichmacher, bevorzugt Ethylphthalat, wobei die Prozentangaben in Gew.-%, bezogen auf das Gewicht der Menge des Beschichtungspolymers, ausgedrückt sind,
• 0 bis 10% eines Permeabilisierungsmittels, bevorzugt kolloidales Siliciumdioxid, wobei die Prozentangaben in Gew.-%, bezogen auf das Gewicht der Menge des Beschichtungspolymers, ausgedrückt sind, und
• 0 bis 10% eines Antistatikmittels, bevorzugt mikronisierter Talk, wobei die Prozentangaben in Gew.-%, bezogen auf das Gewicht der Menge des Beschichtungspolymers, ausgedrückt sind.

7. Granulat nach einem der Ansprüche 1 bis 6, **dadurch gekennzeichnet, dass** es weiterhin einen Überzug umfasst, der im Wesentlichen aus Acrylsäure-Polymer, Zellulose-Polymer, thermoplastischem Polymer oder Gemischen davon, besteht.

8. Verfahren zur Herstellung von Granulat nach einem der Ansprüche 1 bis 7, **dadurch gekennzeichnet, dass** es die aufeinander folgenden Schritte umfasst, bestehend aus:
• trockenes Vermischen der Mikrokristalle des Allylamin- oder Benzylamin-Antimykotikums oder eines pharmazeutisch annehmbaren Salzes davon, gegebenenfalls mit einem Antistatikmittel und/oder einem Verdünnungsmittel und/oder Permeabilisierungsmittel;
• Granulieren des Gemischs, das in dem obigen Schritt erhalten wurde, durch Sprühen einer Lösung oder Suspension des Bindemittels,
• Beschichten des so erhaltenen Granulats mit einer Suspension der Beschichtungszusammensetzung,
• gegebenenfalls Aufbringen eines Überzugs, auf Basis von Acrylsäure-Polymer, Zellulose-Polymer, thermoplastischem Polymer oder Gemischen davon, auf dem so erhaltenen Granulat,
• Trocknen des so erhaltenen beschichteten Granulats.

9. Verfahren nach Anspruch 8, wobei es sich bei der Granulierung um eine Boden-Spühgranulierung, tangentielle Sprühgranulierung, Aufsprüh-Granulierung oder Schnell-Granulierung, bevorzugt Boden-Spühgranulierung, handelt.

10. Orodispersible Tabletten, die beschichtetes Granulat nach einem der Anprüche 1 bis 7 oder nach einem der Ansprüche 8 oder 9 hergestelltes Granulat, und ein Gemisch aus Hilfsstoffen umfassen, das wenigstens ein Zersetzungsmittel, ein lösliches Verdünnungsmittel, ein Gleitmittel und gegebenenfalls ein Quellmittel, ein Permeabilisierungsmittel, Süßstoffe, Geschmacksstoffe und Farbstoffe umfasst.

11. Orodispersible Tabletten nach Anspruch 10, in denen das Mischungsverhältnis der Hilfsstoffe zu dem beschichteten Granulat 0,4 bis 10, bevorzugt 0,4 bis 5 und noch weiter bevorzugt 1 bis 2 Teile pro Gewicht beträgt, wobei das Gemisch aus Hilfsstoffen umfasst:
• wenigstens ein Zersetzungsmittel,
• ein lösliches Verdünnungsmittel das Bindungseigenschaften aufweist,
• ein Gleitmittel,
• ein Permeabilisierungsmittel,
• und gegebenenfalls Süßstoffe, Geschmacksstoffe und Farbstoffe.

12. Orodispersible Tabletten nach Anspruch 10 oder 11, in denen das Zersetzungsmittel ausgewählt ist aus der Gruppe, bestehend aus Croscarmellose, Crospovidon und Gemischen davon.

13. Orodispersible Tabletten nach einem der Ansprüche 10 bis 12, in denen das lösliche Verdünnungsmittel mit Bindungseigenschaften aus einem Polyol mit weniger als 13 Kohlenstoffatomen besteht, das entweder in Form des direkt pressbaren Produkts mit einer mittleren Teilchengröße von 100 bis 500 µm oder in Form eines Pulvers mit einer mittleren Teilchengröße von weniger als 100 µm vorliegt, wobei dieses Polyol bevorzugt ausgewählt ist aus der Gruppe bestehend aus Mannitol, Xylitol, Sorbitol und Maltitol, wobei das Sorbitol nicht allein verwendet werden kann und wobei das lösliche Verdünnungsmittel, wenn nur eines davon verwendet wird, in Form des direkt pressbaren Produkts verwendet wird, wohingegen dann, wenn wenigstens zwei lösliche Verdünnungsmittel mit Bindungseigenschaften vorhanden sind, eines in der direkt pressbaren Form vorliegt und das andere in Pulverform vorliegt, wobei es dann möglich ist, dass es sich bei den Polyolen um dasselbe Polyol handelt, wobei das Verhältnis des direkt pressbaren Polyols zu dem pulverförmigen Polyol 99/1 bis 20/80, bevorzugt 80/20 bis 20/80 beträgt.

14. Orodispersible Tabletten nach einem der Ansprüche 10 bis 13, in denen das Permeabilisierungsmittel ausgewählt ist aus der Gruppe umfassend insbesondere Siliziumdioxide mit einer hohen Affinität für wässrige Lösungsmittel, wie beispielsweise präzipitiertes Siliziumdioxid, besser bekannt unter der Handelsmarke Syloid^{®}, Maldodextrine, β-Cyclodextrine, und Gemische davon.

15. Orodispersible Tabletten nach einem der Ansprüche 10 bis 14, in denen das Gleitmittel ausgewählt ist aus der Gruppe, bestehend aus Magnesiumstearat, Stearinsäure, Natriumstearylfumarat, mikronisiertem Polyoxyethylenglykol (mikronisiertes Macrogol 6000), Leucin, Natriumbenzoat und Gemischen davon.

16. Orodispersible Tabletten nach einem der Ansprüche 10 bis 15, in denen der Anteil an Zersetzungsmittel 1 bis 20 Gew.%, bevorzugt 5 bis 15 Gew.-%, beträgt, und der Anteil des löslichen Mittels 30 bis 90 Gew.%, bevorzugt 30 bis 50 Gew.-% beträgt, jeweils bezogen auf das Gewicht der Tablette.

17. Verwendung von Granulat von Allylamin- oder Benzylamin-Antimykotika oder eines pharmazeutisch annehmbaren Salzes davon, nach einem der Ansprüche 1 bis 7, zur Herstellung von orodispersiblen Tabletten nach einem der Ansprüche 10 bis 16, für die folgenden therapeutischen Anwendungen: Onychomycose, hervorgerufen durch Dermatophyt-Pilze, Tinea capitis, Pilzinfektionen der Haut, für die Behandlung von Tinea corporis, Tinea cruris, Tinea pedis und Hefeinfektionen der Haut, hervorgerufen durch die Gattung Candida, z.B. Candida albicans, systemische Mykose, Mykose durch Azolresistente Stämme, z.B. in Kombination mit einem 14-alpha-Methyldimethylase-Inhibitor, oder Infektionen mit Helicobakter pylori.

## Revendications

1. Granules d'un antimycosique allylamine ou benzylamine ou de l'un de ses sels pharmaceutiquement acceptables, **caractérisés en ce que** les granules sont enrobés et qu'ils contiennent :
- des microcristaux d'un antimycosique allylamine ou benzylamine ou de l'un de ses sels pharmaceutiquement acceptables,
- au moins un liant choisi dans le groupe consistant en des polymères cellulosiques tels que l'éthylcellulose, l'hydroxypropylcellulose et l'hydroxypropylméthylcellulose, des polymères acryliques tels qu'un copolymère acrylate ammoniométhacrylate insoluble, un polyacrylate ou un copolymère polyméthacrylique ; des povidones, des alcools polyvinyliques et des mélanges de ceux-ci,
- facultativement un agent diluant choisi dans le groupe comprenant des dérivés cellulosiques, une cellulose microcristalline, des amidons, le lactose, des polyols tels que le mannitol et/ou un agent antistatique choisi dans le groupe comprenant le talc micronisé ou non micronisé et/ou un agent perméabilisant choisi dans le groupe consistant en la silice colloïdale et la silice précipitée.

2. Granules selon la revendication 1, **caractérisés en ce que** l'antimycosique allylamine ou benzylamine est choisi dans le groupe consistant en la buténafine, la naftifine et de préférence la terbinafine.

3. Granules selon la revendication 1 ou la revendication 2, **caractérisés en ce qu'**ils présentent une répartition granulométrique telle qu'au moins 50 %, de préférence au moins 70 %, des granules ont une taille de particules comprise entre 125 et 355 µm et que moins de 15 % des granules ont une taille de particules inférieure à 90 µm.

4. Granules selon l'une quelconque des revendications 1 à 3, **caractérisés en ce qu'**ils comprennent :
- de 10 % à 80 % de granules de l'antimycosique allylamine ou benzylamine ou d'un de ses sels pharmaceutiquement acceptables, par rapport au poids des granules de l'antimycosique allylamine ou benzylamine ou d'un de ses sels pharmaceutiquement acceptables,
- au plus 15 %, de préférence au plus 5 % en poids du liant, par rapport au poids de l'antimycosique allylamine ou benzylamine ou d'un de ses sels pharmaceutiquement acceptables,
- au plus 2 %, de préférence 0,5 % en poids de l'agent antistatique, par rapport au poids des granules de l'antimycosique allylamine ou benzylamine ou d'un de ses sels pharmaceutiquement acceptables.

5. Granules selon l'une quelconque des revendications 1 à 4, **caractérisés par le fait qu'**ils sont enrobés d'une composition d'enrobage contenant au moins un polymère d'enrobage choisi dans le groupe consistant en des polymères cellulosiques tels que l'éthylcellulose, l'hydroxypropylcellulose et l'hydroxypropylméthylcellulose, des polymères acryliques tels qu'un copolymère acrylate ammonio-méthacrylate insoluble, un polyacrylate, ou des copolymères méthacryliques utilisés seuls, en combinaison ou en mélange avec des polymères dépendants du pH, et leurs mélanges, et facultativement un agent perméabilisant, des plastifiants et des agents solubles tels que des polyols.

6. Granules selon l'une quelconque des revendications 1 à 5, **caractérisés par le fait qu'**ils comprennent :
- de 10 % à 95 % de granules d'un antimycosique allylamine ou benzylamine ou de l'un de ses sels pharmaceutiquement acceptables, de préférence la terbinafine HCl, les pourcentages étant exprimés en poids par rapport au poids des granules de l'antimycosique allylamine ou benzylamine ou de l'un de sels pharmaceutiquement acceptables,
- de 5 à 60 %, de préférence de 5 à 40 % et de manière encore préférable de 10 à 20 % d'un polymère d'enrobage, les pourcentages étant exprimés en poids par rapport au poids des granules de l'antimycosique allylamine ou benzylamine ou de l'un de sels pharmaceutiquement acceptables,
- de 0 à 30 %, de préférence 10 % en poids de plastifiants, de préférence du phtalate d'éthyle, les pourcentages étant exprimés en poids par rapport au poids de la quantité du polymère d'enrobage,
- de 0 à 10 % d'un agent perméabilisant, de préférence de la silice colloïdale, les pourcentages étant exprimés en poids par rapport au poids de la quantité du polymère d'enrobage, et
- de 0 à 10 % d'un agent antistatique, de préférence du talc micronisé, les pourcentages étant exprimés en poids par rapport au poids de la quantité du polymère d'enrobage.

7. Granules selon l'une quelconque des revendications 1 à 6, **caractérisés par le fait qu'**ils comprennent en outre un sur-enrobage consistant essentiellement en un polymère acrylique, un polymère cellulosique, un polymère thermoplastique ou des mélanges de ceux-ci.

8. Procédé de préparation de granules selon l'une quelconque des revendications 1 à 7, **caractérisé par le fait qu'**il comprend les étapes successives consistant à :
- mélanger à sec les microcristaux de l'antimycosique allylamine ou benzylamine ou de l'un de ses sels pharmaceutiquement acceptables facultativement avec un agent antistatique et/ou un agent diluant et/ou un agent perméabilisant ;
- transformer en granules le mélange obtenu à l'étape ci-dessus en pulvérisant une solution ou une suspension du liant,
- enrober les granules ainsi obtenus avec suspension de la composition d'enrobage,
- facultativement, appliquer sur les granules enrobés ainsi obtenus un sur-enrobage à base d'un polymère acrylique, d'un polymère cellulosique, d'un polymère thermoplastique ou des mélanges de ceux-ci,
- sécher les granules enrobés ainsi obtenus.

9. Procédé selon la revendication 8, dans lequel la granulation est une granulation par pulvérisation par le fond, une granulation par pulvérisation tangentielle, une granulation par pulvérisation par le haut ou une granulation à cisaillement élevé, de préférence une granulation par pulvérisation par le fond.

10. Comprimés orodispersibles comprenant des granules enrobés selon l'une quelconque des revendications 1 à 7 ou préparés selon la revendication 8 ou 9 et un mélange d'excipients comprenant au moins un agent désintégrant, un agent diluant soluble, un lubrifiant et facultativement un agent gonflant, un agent perméabilisant, des édulcorants, des aromatisants et des colorants.

11. Comprimés orodispersibles selon la revendication 10, dans lesquels le rapport entre le mélange d'excipients et les granules enrobés est de 0,4 à 10, de préférence de 0,4 à 5 et de manière encore préférable de 1 à 2 parties en poids, le mélange d'excipients comprenant :
- au moins un agent désintégrant,
- un agent diluant soluble qui présente des propriétés liantes,
- un lubrifiant,
- un agent perméabilisant,
- et facultativement des édulcorants, des aromatisants et des colorants.

12. Comprimés orodispersibles selon la revendication 10 ou 11, dans lesquels l'agent désintégrant est choisi dans le groupe consistant en la croscarmellose, la crospovidone et des mélanges de celles-ci.

13. Comprimés orodispersibles selon l'une quelconque des revendications 10 à 12, dans lesquels l'agent diluant soluble à propriétés liantes consiste en un polyol ayant moins de 13 atomes de carbone et étant soit sous forme du produit directement compressible avec une taille de particules moyenne de 100 à 500 µm, soit sous forme d'une poudre avec une taille de particules moyenne inférieure à 100 µm, ce polyol de préférence étant choisi dans le groupe consistant en le mannitol, le xylitol, le sorbitol et le maltitol, étant entendu que le sorbitol ne peut pas être utilisé seul et que, dans le cas où il n'y a qu'un seul agent diluant soluble à propriétés liantes, il est utilisé sous forme du produit directement compressible, tandis que dans le cas où il y a au moins deux agents diluants solubles à propriétés liantes, l'un est présent sous forme directement compressible et l'autre est présent sous forme de poudre, les polyols pouvant alors être les mêmes, le rapport entre le polyol directement compressible et le polyol en poudre étant de 99/1 à 20/80, de préférence de 80/20 à 20/80.

14. Comprimés orodispersibles selon l'une quelconque des revendications 10 à 13, dans lesquels l'agent perméabilisant est choisi dans le groupe comprenant en particulier des silices présentant une affinité élevée pour les solvants aqueux, telles que la silice précipitée mieux connue sous la marque commerciale Syloïd®, les maltodextrines, les β-cyclodextrines et des mélanges de celles-ci.

15. Comprimés orodispersibles selon l'une quelconque des revendications 10 à 14, dans lesquels le lubrifiant est choisi dans le groupe consistant en le stéarate de magnésium, l'acide stéarique, le stéaryl-fumarate de sodium, le polyoxyéthylène glycol micronisé (Macrogol 6000 micronisé), la leukine, le benzoate de sodium et des mélanges de ceux-ci.

16. Comprimés orodispersibles selon l'une quelconque des revendications 10 à 15, dans lesquels la proportion d'agent désintégrant est de 1 à 20 % en poids, de préférence de 5 à 15 % en poids, et la proportion d'agent soluble est de 30 à 90 % en poids, de préférence de 30 à 50 % en poids, dans chaque cas sur la base du poids du comprimé.

17. Utilisation de granules d'un antimycosique allylamine ou benzylamine ou de l'un de ses sels pharmaceutiquement acceptables selon l'une quelconque des revendications 1 à 7 pour la fabrication de comprimés orodispersibles selon l'une quelconque des revendications 10 à 16 pour les applications thérapeutiques suivantes : une onychomycose causée par un champignon dermatophyte, une teigne tondante microscopique, des infections fongiques de la peau, pour le traitement d'une trichophytie de la peau glabre, un eczéma marginé, une épidermophytie et des infections à levure de la peau causées par le genre Candida, par exemple Candida albicans, une mycose profonde, une mycose par des souches résistantes à l'azole, par exemple en combinaison avec un inhibiteur de la 14-alpha-méthyldiméthylase, ou des infections à Helicobacter pylori.
